# EUROPÄISCHE PATENTANMELDUNG

(11) **EP 0 780 355 A1**
(43) Veröffentlichungstag der Anmeldung: **25.06.1997**
(21) Anmeldenummer: 96120154.8
(22) Anmeldetag: 16.12.1996
(51) Int. Cl.: C07C 17/14

(54) **Verfahren zur Herstellung von aromatischen (Bromalkyl)-substituierten Kohlenwasserstoffverbindungen**

(30) Priorität: 18.12.1995 DE 19547249
(71) Anmelder: HOECHST AKTIENGESELLSCHAFT, 65929 Frankfurt am Main (DE)
(72) Erfinder: Millauer, Hans, Dr., 65760 Eschborn (DE); Küber, Frank, Dr., 61440 Oberursel (DE)

(57) **Zusammenfassung**

Die vorliegende Erfindung betrifft Verfahren zur Herstellung einer aromatischen (Bromalkyl)-substituierten Kohlenwasserstoffverbindung wobei eine alkylsubstituierte aromatische Kohlenwasserstoffverbindung mit einem Bromierungsmittel in Gegenwart von Wasser umgesetzt wird.

## Beschreibung

Die vorliegende Erfindung betrifft ein Verfahren zur Herstellung von aromatischen (Bromalkyl)-substituierten Kohlenwasserstoffverbindungen durch Bromierung einer alkylsubstituierten Kohlenwasserstoffverbindung mit einem Bromierungsmittel in Anwesenheit von Wasser und gegebenenfalls eines organischen Lösemittels sowie gegebenenfalls eines Reduktionsmittels.

Aromatische (Bromalkyl)-substituierte Kohlenwasserstoffverbindungen sind wichtige Zwischenprodukte bei der Synthese organischer Verbindungen, insbesondere bei der Herstellung von Wirkstoffen für Pharmazeutische Produkte, Pflanzenschutzmittel und zahlreiche andere Verwendungszwecke.

So ist zum Beispiel 2,2'-Bis-(brommethyl)-1,1'-binaphthyl ein wertvolles Zwischenprodukt für die Herstellung einer Reihe von bifunktionellen organischen Verbindungen, die sich durch den Austausch der Bromatome beispielsweise in Alkohole, Ester, Amine, quartäre Ammoniumsalze, Phosphine oder Phosphinigsäureester überführen lassen. Zum Beispiel stellt 2,2'-Bis-(brommethyl)-1,1'-binaphthyl ein zentrales Zwischenprodukt bei der Herstellung von 2,2'-Bis-[(diphenylphosphino)-methyl]-1,1'-binaphthyl (NAPHOS) und weiteren bidentalen Phosphorliganden dar. Gemäß der JP-A 79/39059 erhält man NAPHOS durch Umsetzung von 2,2'-Bis-(brommethyl)-1,1'-binaphthyl mit Diphenylphosphinigsäuremethylester und anschließende Reduktion mit Trichlorsilan. Gemäß den deutschen Patentanmeldungen EP-A-684 248 und EP-A-685 456 erfolgt die Herstellung von NAPHOS ausgehend von 2,2'-Bis-(brommethyl)-1,1'-binaphthyl durch zweifache Quaternierung mit Trimethylamin und nachfolgende Umsetzung mit Magnesium-diphenyl-phosphid.
NAPHOS oder daraus hergestellte wasserlösliche Polysulfonate (BINAS) können als Liganden zur Herstellung von Komplexen bestimmter Schwermetallatome verwendet werden. Wasserlösliche Komplexe von BINAS mit Rhodium beispielsweise werden als Katalysatoren für den großtechnisch durchgeführten Prozeß der Hydroformylierung von Olefinen, insbesondere von Propylen ("Oxosynthese") eingesetzt.

Eine weitere, technisch bedeutsame Brommethylverbindung ist 2-(Brommethyl)biphenyl, welches zur Synthese von Indenverbindungen benötigt wird.
2-Methylinden beispielsweise dient zum Aufbau von verbrückten Liganden für neuartige Metallocene des Zirkons, einer hochwirksamen Klasse von Katalysatoren für die Olefinpolymerisation.

Methylsubstituierte aromatische Kohlenwasserstoffe lassen sich an der Methylgruppe mit N-Bromsuccinimid selektiv bromieren. Zahlreiche derartige Verfahren sind beschrieben. Eine Zusammenfassung befindet sich in "Methoden der Organischen Chemie" (Houben-Weyl), 4.Auflage, Band V/4, Seiten 341 ff.
Als Lösemittel werden hauptsächlich Tetrachlorkohlenstoff oder andere chlorierte Lösemittel verwendet.
Die Herstellung von 2,2'-Bis-(brommethyl)-1,1'-binaphthyl ist ebenfalls mehrfach beschrieben worden. Sie erfolgt durch Bromierung von 2,2'-Dimethyl-1,1'-binaphthyl mit N-Bromsuccinimid in Gegenwart eines Radikalbildners und/oder unter Bestrahlung mit kurzwelligem Licht mittels einer UV-Lampe. Als Lösemittel werden dabei Verbindungen eingesetzt, die unter den Reaktionsbedingungen inert sind und die Kernbromierung unterdrücken, wie Tetrachlorkohlenstoff (M. E. Jung et al., Tetrahedron Letters 29 (1988) 6199; H. J. Bestmann et al., Chem. Ber. 107 (1974) 2926; P. Mazaleyrat, Chem. Commun. 1985, 317; T. Hayashi et al., J. Am. Chem. Soc. 110 (1988) 8153) oder Chlorbenzol bzw. Dichlorbenzol (EP-A-675 095).

Bisher ist bei Bromierungen von alkylsubstituierten aromatischen Kohlenwasserstoffverbindung die Gegenwart von unpolaren Solventien empfohlen worden (J. March, Advanced Org. Chemistry, 4th Ed., 1992, Seite 695). Ein Nachteil dieser Verfahren besteht darin, daß während der Bromierung entstehende Nebenprodukte, z.B. Succinimid als Feststoff anfallen und sich an den kühleren Teilen der Apparatur, z.B. an den Oberflächen von Wärmetauschern absetzen. Dadurch wird die Wärmeabfuhr aus dem Reaktionsmedium behindert, was zu unerwünschtem Temperaturanstieg führen kann.
Bei licht-initiierten Verfahren kann es zusätzlich infolge mangelnder Wärmeabfuhr zur Bildung von Belägen auf dem Tauchrohr der UV-Lampe und zu einer Störung des Lichteintrags kommen.
Bei der Übertragung der im Stand der Technik beschriebenen Verfahren in den technischen Maßstab tritt häufig noch ein weiterer Mangel zutage: Beim Einsatz von im halbtechnischen oder technischen Maßstab hergestellten Ausgangsverbindungen (technical grade) wird die Bromierung mit N-Bromsuccinimid bisweilen erheblich gehemmt. insbesondere erfordert die Bromierung der Verbindungen 2,2'-Dimethyl-1,1'- binaphthyl oder 2-Methylbiphenyl, die aufgrund ihrer üblichen Herstellweise (reduktive Kupplung einer aromatischen Grignardververbindung mit einer aromatischen Bromverbindung unter katalytischen Zusätzen von bestimmten Metallverbindungen) noch Spuren dieser Metalle wie Palladium oder Nickel enthalten, häufig sehr lange Reaktionszeiten. So beobachtet man beispielsweise, daß im Falle von 2,2'-Dimethyl-1,1'- binaphthyl nach der Zugabe des Bromierungsmittels zunächst eine längere Induktionsperiode eintritt bis die Bromierungsreaktion anspringt. Auch im weiteren Verlauf der Bromierung kann es zu einer starken Verzögerung der Reaktion kommen, woraus Reaktionszeiten von 5 bis 8 Stunden oder länger resultieren (EP-A-675 095).
Auch 2,2'-Dimethyl-1,1'-binaphthyl, welches alternativ durch oxidative Kupplung nach dem elektrochemischen Verfahren gemäß EP-A-0 663 378 gewonnen wird, kann herstellungsbedingt noch stark inhibierende Stoffe enthalten.
Die üblicherweise durchgeführten Maßnahmen wie hohe Bestrahlungsleistungen oder höhere Konzentrationen an Radikalbildnern, größere Überschüsse an Bromierungsmittel (z.B. N-Bromsuccinimid) und/oder lange Reaktionszeiten vermögen das Problem der Inhibierung nur partiell zu lösen ohne dessen Ursachen zu beseitigen.

Die geschilderten Verfahren sind aus diesen Gründen für eine wirtschaftliche Betriebsweise in technischem Maßstab nicht geeignet.

Es war wünschenswert, ein Verfahren zu entwickeln, welches technische Probleme während des Prozesses und Reaktionsverzögerungen vermeidet.

Es bestand daher die Aufgabe ein Verfahren zur Verfügung zu stellen, nach welchem aromatische (Bromalkyl)-substituierte Kohlenwasserstoffverbindungen in industriellem Maßstab, mit hohen Raum-Zeit-Ausbeuten und unter hohen Sicherheitsstandards hergestellt werden können. Diese Aufgabe wird durch das erfindungsgemäße Verfahren gelöst.

Die vorliegende Erfindung betrifft somit ein Verfahren zur Herstellung einer aromatischen (Bromalkyl)-substituierten Kohlenwasserstoffverbindung, wobei eine alkylsubstituierte aromatische Kohlenwasserstoffverbindung mit einem Bromierungsmittel in Gegenwart von Wasser umgesetzt wird.

Beispiele für nach dem erfindungsgemäßen Verfahren erhältliche aromatische (Bromalkyl)-substituierte Kohlenwasserstoffverbindungen sind ein- oder mehrkernige Aromaten, die einen oder mehrere (Bromalkyl)-Substituenten tragen. Die Bromalkylsubstituenten weisen bevorzugt 1 bis 10 Kohlenstoffatome und eines oder mehrere Bromatome auf. Besonders bevorzugt ist Brommethyl. Bromatome können auch an zwei Alkylsubstituenten sitzen, die miteinander zu einem Ring verbunden sind. Beispiele für aromatische (Bromalkyl)-substituierte Kohlenwasserstoffverbindungen sind 2,2'-Bis-(brommethyl)-1,1'-binaphthyl, 2-(Brommethyl)-biphenyl, 1,2-Dibromacenaphthen, o-(Brommethyl)-toluol, m-(Brommethyl)-toluol, p-(Brommethyl)-toluol, Benzylbromid, Benzalbromid, o-Chlorbenzylbromid, m-Chlorbenzylbromid, p-Chlorbenzylbromid, o-Brombenzylbromid, m-Brombenzylbromid oder p-Brombenzylbromid.

Unter dem Begriff alkylsubstituierte aromatische Kohlenwasserstoffverbindung werden z.B. ein- oder mehrkernige Aromaten verstanden, die einen oder mehrere Alkylsubstituenten tragen. Die Alkylsubstituenten weisen bevorzugt 1 bis 10 Kohlenstoffatome auf, besonders bevorzugt ist Methyl. Es können auch zwei Alkylsubstituenten miteinander zu einem Ring verbunden sein. Die alkylsubstituierte aromatische Kohlenwasserstoffverbindung kann neben den Alkylsubstituenten auch weitere Substituenten wie Halogenatome tragen.
Beispiele für alkylsubstituierte aromatische Kohlenwasserstoffverbindungen sind Toluol, o-Xylol, m-Xylol, p-Xylol, 2,2'-Dimethyl-1,1'-binaphthyl, 2-Methylbiphenyl, Acenaphten, o-Chlortoluol, m-Chlortoluol, p-Chlortoluol, o-Bromtoluol, m-Bromtoluol oder p-Bromtoluol.

Als Bromierungsmittel werden alle Verbindungen verstanden, die in der Lage sind, alkylsubstituierte aromatische Kohlenwasserstoffverbindungen zu bromieren. Bevorzugt sind N-Bromsuccinimid, N-Bromphthalimid oder 1,3-Dibrom-5,5-dimethyl-hydantoin.

Werden als Ausgangsstoffe Verbindungen eingesetzt, die bei der gewählten Reaktionstemperatur nicht flüssig sind oder deren Bromierungsprodukte bei der gewählten Reaktionstemperatur nicht flüssig sind, wird das erfindungsgemäße Verfahren bevorzugt in Anwesenheit eines organischen Lösemittels durchgeführt.

Als organische Lösungsmittel sind solche bevorzugt, die unter Reaktionsbedingungen inert oder weitgehend inert sind und sich mit Wasser nicht oder nur in geringem Ausmaß mischen, d.h. Verbindungen, die einen unpolaren oder nur wenig polaren Charakter besitzen. Üblicherweise werden als organische Lösemittel ein- oder mehrfach halogenierte, insbesondere chlorierte, aliphatische oder aromatische Kohlenwasserstoffe eingesetzt wie Methylenchlorid, Chloroform, Tetrachlorkohlenstoff, 1,2-Dichlorethan, Chlorbenzol, 1,2-, 1,3- oder 1,4-Dichlorbenzol oder Mischungen der genannten Lösemittel verwendet. Ferner können als organische Lösemittel auch Fluorchlorkohlenwaserstoffe wie 1,1,2-Trichlor-trifluorethan verwendet werden.

Falls ein organisches Lösemittel eingesetzt wird, ist die Untergrenze der Menge bevorzugt so bemessen, daß sowohl die umzusetzende alkylsubstituierte aromatische Kohlenwasserstoffverbindung als auch das daraus erhaltene Bromierungsprodukt sich darin vollständig lösen. Die Obergrenze ist nicht kritisch. Im allgemeinen genügt es, ein Gewichtsverhältnis der alkylsubstituierten aromatischen Kohlenwasserstoffverbindung zum organischen Lösemittel von etwa 1 : (1 bis 20), bevorzugt 1 : (3 bis 10) zu verwenden.

Die Mindestmenge des in dem erfindungsgemäßen Verfahren eingesetzten Wassers ist so bemessen, daß gebildete Nebenprodukte (z.B. Succinimid) darin entweder vollständig oder in einem solchen Ausmaß gelöst werden, so daß es durch den ungelösten Anteil zu keiner Beeinträchtigung des Reaktionsverlaufes kommen kann. Im allgemeinen beträgt das Gewichtsverhältnis des Bromierungsmittels (z.B. N-Bromsuccinimid) zu Wasser etwa 1 : (1 bis 20), bevorzugt 1 : (1,5 bis 5).

In einer bevorzugten Aufführungsform des erfindungsgemäßen Verfahrens wird dem Reaktionsgemisch ein Reduktionsmittel zugesetzt. Beispiele für Reduktionsmittel sind reduzierende Verbindungen des Schwefels oder Phosphor in seinen niedrigen Oxidationsstufen, beispielsweise Salze wie Sulfiten, Hydrogensulfiten, Bisulfiten, Dithioniten, Thiosulfaten, Hypophosphiten oder Phosphiten; ferner eignen sich reduzierende Stickstoffverbindungen, beispielsweise Hydroxylamin oder Hydrazin. Ebenfalls geeignet sind reduzierende Metallionen, beispielsweise Eisen(II)- oder Titan(III)-Verbindungen.
Das Molverhältnis des Reduktionsmittels zu der alkylsubstituierten aromatischen Kohlenwasserstoffverbindung beträgt (0,001 bis 0,1) : 1, vorzugsweise (0,005 bis 0,05) : 1.

Das erfindungsgemäße Verfahren wird bevorzugt unter Bestrahlung mit kurzwelligem Licht und/oder in Anwesenheit eines Radikalbildners durchgeführt.
Als Lichtquellen können übliche Strahlungsquellen dienen, mit einem ausreichenden Anteil an kurzwelligem Tageslicht oder ultravioletter Strahlung, insbesondere im Spektralbereich von etwa 250 bis 500 Nanometer.

Falls das erfindungsgemäße Verfahren in Gegenwart eines Radikalbildners durchgeführt wird, können übliche Radikalbildner wie Peroxoverbindungen, beispielsweise Dialkylperoxide, Diacylperoxide, Alkylhydroperoxide, Percarbonsäuren, Peroxoverbindungen anorganischer Säuren oder organische Azoverbindungen eingesetzt werden.
Die gegebenenfalls zugesetzten Radikalbildner werden in den üblichen Gewichtsverhältnissen von etwa 0,1 bis 5, vorzugsweise 0,5 bis 2 % bezogen auf das Edukt zugesetzt. Auch die Kombination aus Bestrahlung und Zusatz von Radikalbildner läßt sich in den erfindundungsgemäßen Verfahren anwenden.

Das erfindungsgemäße Verfahren wird bei Temperaturen von -10°C bis 100°C, vorzugsweise von 10°C bis 70°C und besonders bevorzugt von 20°C bis 50°C durchgeführt.

Falls in dem erfindungsgemäßen Verfahren ein organisches Lösemittel eingesetzt wurde, ist es bevorzugt, zur Aufarbeitung des Reaktionsgemisches die wäßrige Phase von der organischen Phase zu trennen. Dazu kann es zweckmäßig sein, vorher die Temperatur des Reaktionsgemisches noch weiter zu erhöhen, um etwaige Mengen suspendierten Reaktionsproukts (z.B. Succinimid) in Lösung zu bringen. Die weiteren Aufarbeitungsschritte verlaufen dann in an sich bekannter Weise, beispielsweise durch Abdestillieren des Lösemittels und anschließendes Destillieren oder Umkristallisieren des Produktes.

Die Bromierungsreaktion nach dem erfindungsgemäßen Verfahren verläuft zügig und ohne nennenswerte Nebenreaktionen. Zur Einführung eines Bromatoms wird pro Mol Edukt etwa ein Mol Bromierungsmittel (z.B. N-Bromsuccinimid) benötigt. Es werden daher bei Monobromierungen das Edukt und das Bromierungsmittel (z.B. N-Bromsuccinimid) im Molverhältnis von 1 : (0,8 bis 1,2), vorzugsweise 1 : (0,9 bis 1,1) und besonders bevorzugt von 1 : (0,95 bis 1,05) eingesetzt. Bei Dibromierungen kommen Edukt und das Bromierungsmittel (z.B. N-Bromsuccinimid) im Molverhältnis von 1 : (1,6 bis 2,4), vorzugsweise 1 : (1,8 bis 2,2) und besonders bevorzugt von 1 : (1,9 bis 2,1) zum Einsatz.

Das erfindungsgemäße Verfahren kann so durchgeführt werden, daß die alkylsubstituierte aromatische Kohlenwasserstoffverbindung und Wasser sowie gegebenenfalls ein organisches Lösemittel und/oder ein Reduktionsmittel vorlegt und durch Rühren miteinander vermischt werden. Zu diesem Gemisch kann das Bromierungsmittel, z.B. N-Bromsuccinimid auf einmal oder in Portionen zugegeben werden. Die Bromierung kann gestartet werden, indem das Reaktionsgemisch mit einer geeigneten Lichtquelle bestrahlt wird und/oder ein Radikalbildner zugegeben wird und das Gemisch auf die Reaktionstemperatur gebracht wird.

Im Falle einer portionsweisen Zugabe des Bromierungsmittels (z.B. N-Bromsuccinimid) wird die Dosiergeschwindigkeit dem Reaktionsverlauf angepaßt. Hinweise auf den Reaktionsverlauf erhält man beispielsweise durch Ermittlung des Temperaturverlaufes oder durch die analytische Erfassung der aktuellen Konzentration des Bromierungsmittels (z.B. N-Bromsuccinimid).

Das erfindungsgemäße Verfahren erlaubt die Herstellung von aromatischen (Bromalkyl)-substituierten Kohlenwasserstoffverbindungen, insbesondere mit erhöhter Raum-Zeit-Ausbeute. Die nachfolgenden Beispiele dienen der Illustration der Erfindung haben jedoch keinerlei limitierenden Charakter.

### Beispiel 1

Man benutzt ein zylindrisches 2 Liter Glasgefäß mit einem gläsernen Kühlmantel und einem Bodenablaßhahn. Das Gefäß ist mit einem Rührer, einem Einfüllstutzen zum Eintrag von N-Bromsuccinimid und einem Thermoelement, verbunden mit digitaler Anzeige (Empfindlichkeit 0,1 °C) und Temperaturschreiber, ausgestattet. Der Kühlmantel ist an einen Thermostat mit Wasser angeschlossen, welches mit einer Konstanz von + /- 0.1°C temperiert werden kann. Die Bestrahlung erfolgt von außen durch den Glasmantel des Reaktors mittels einer Tageslichtlampe ("VITALUX", 300 Watt), welche 60 mm vom Glasmantel des Reaktors positioniert ist.
Im Reaktor legt man 212 g (0,75 Mol) 2,2'-Dimethyl-1,1'-binaphthyl, 900 ml Chlorbenzol, 450 ml Wasser, 68 g (0,38 Mol) N-Bromsuccinimid und 0,52 g (0,005 Mol) Natriumhydrogensulfit vor.
Das Gemisch wird unter kräftigem Rühren auf eine Temperatur von 19-20°C gebracht (die Temperatur der Kühlsole beträgt während der gesamten Versuchsdauer konstant 18,0°C). Die Luft wird durch Einleiten und Überlagern mit Stickstoff aus dem Reaktor verdrängt und die Bestrahlung in Gang gesetzt.
Nach einer Induktionsperiode von etwa 5 bis 10 Minuten setzt die Reaktion ein, wobei sich die Temperatur um etwa 5° erhöht. Unmittelbar nachdem die Temperatur wieder zu fallen beginnt, werden weitere 68 g N-Bromsuccinimid eingetragen. Nachdem man wiederum das Erreichen des Temperaturmaximums abgewartet hat, werden weitere 67 g N-Bromsuccinimid, im Anschluß daran weitere Portionen von 34 g, dann 30 g und schließlich 8 g N-Bromsuccinimid eingetragen. Die Zugabe der letzten Portion erfolgt durchschnittlich etwa 1 Stunde nach Beginn der Bestrahlung. Insgesamt werden 275 g (1,55 Mol) N-Bromsuccinimid eingesetzt. Nach insgesamt 2 Stunden wird die Bestrahlung beendet. Das Reaktionsgemisch wird anschließend auf 40°C erwärmt und die beiden flüssigen Phasen geschieden. Die Chlorbenzol-Phase wird einmal mit 450 ml Wasser gewaschen und anschließend unter vermindertem Druck eingedampft. Der Destillationsrückstand, welcher aus einem bräunlichen Feststoff besteht, wird mit 800 ml heißem Methanol verrührt und abgesaugt, der Filterkuchen mit 200 ml Methanol gewaschen und getrocknet. Die Ausbeute beträgt 290 g (88 %) 2,2'-Bis-(brommethyl)-1,1'-binaphthyl. Die HPLC- Analyse (Säule: Hypersil; Eluent: n-Hexan/Methylenchlorid 96:4; Flow: 1,5 ml/Min.) ergibt folgende Zusammensetzung des Produktes: 84 % 2,2'-Bis-(brommethyl)-1,1'-binaphthyl, 4 % 2-(Brommethyl)- 2'-methyl-1,1'-binaphthyl, 10 % 2-(Brommethyl)-2'-(dibrommethyl)-1,1'-binaphthyl, 2 % Unbekannte.

### Beispiel 2

In einer Versuchsapparatur wie in Beispiel 1 beschrieben werden 336 g (2,0 Mol) 2-Methyl-biphenyl (technisch, Reinheit 89 %), 700 ml Chlorbenzol, 600 ml Wasser, 366 g (1,03 Mol) N-Bromsuccinimid und 1,9 g (0,01 Mol) Natriumpyrosulfit, Na₂S₂O₅ vorgelegt.
Das Gemisch wird unter kräftigem Rühren auf eine Temperatur von 24-25°C gebracht (die Temperatur der Kühlsole beträgt während der gesamten Versuchsdauer konstant 23,0°C). Die Luft wird durch Einleiten und Überlagern mit Stickstoff aus dem Reaktor verdrängt und die Bestrahlung in Gang gesetzt.
Nach einer Induktionsperiode von etwa 20 Minuten setzt die Reaktion ein, wobei sich die Temperatur um etwa 3-4° erhöht. . Nach insgesamt 3 Stunden wird die Bestrahlung beendet. Das Reaktionsgemisch wird anschließend auf 35°C erwärmt und die beiden flüssigen Phasen geschieden. Die Chlorbenzol-Phase wird einmal mit 600 ml Wasser gewaschen und anschließend unter vermindertem Druck eingedampft. Man erhält ein hellbraunes Öl, welches nach fraktionierender Destillation 425 g 2-(Brommethyl)- biphenyl, Kp. 128-134°C/0,03 mbar liefert. Die Ausbeute beträgt 86 %. Gemäß GC-Analyse (Gerät: Hewlett Packard 5890 Series II; Säule: 30 m HP1; 80°C (2 Min.), 10°/Min., 270°C (15 Min.); Flow: He, 1,0 ml/Min.) beträgt die Reinheit des Produktes 89,5 %.

## Patentansprüche

1. Verfahren zur Herstellung einer aromatischen (Bromalkyl)-substituierten Kohlenwasserstoffverbindung wobei eine alkylsubstituierte aromatische Kohlenwasserstoffverbindung mit einem Bromierungsmittel in Gegenwart von Wasser umgesetzt wird.

2. Verfahren gemäß Anspruch 1, worin das Reaktionsgemisch zusätzlich ein organisches Lösemittel enthält.

3. Verfahren gemäß Anspruch 1 oder 2, worin das Reaktionsgemisch zusätzlich ein Reduktionsmittel enthält.

4. Verfahren gemäß einem oder mehreren der Ansprüche 1 bis 3, worin das Reaktionsgemisch zusätzlich einen Radikalstarter enthält.

5. Verfahren gemäß einem oder mehreren der Ansprüche 1 bis 4, worin das Reaktionsgemisch mit Licht bestrahlt wird.

6. Verfahren gemäß einem oder mehreren der Ansprüche 1 bis 5, worin das Bromierungsmittel N-Bromsuccinimid ist.

7. Verwendung von Wasser bei der Herstellung einer aromatischen (Bromalkyl)-substituierten Kohlenwasserstoffverbindung.
